# EUROPÄISCHE PATENTSCHRIFT

(11) **EP 3 082 745 B1**
(45) Veröffentlichungstag und Bekanntmachung des Hinweises auf die Patenterteilung: **31.07.2019**
(21) Anmeldenummer: 14799134.3
(22) Anmeldetag: 12.11.2014
(51) Int. Cl.: A61K 8/39, A61Q 5/06, A61K 8/04

(54) **PFLEGENDES TREIBMITTELHALTIGES HAARKOSMETIKUM**
BLOWING AGENT-CONTAINING HAIR CARE COSMETIC
PRODUIT COSMÉTIQUE DE SOIN CAPILLAIRE CONTENANT UN AGENT PROPULSEUR

(30) Priorität: 20.12.2013 DE 102013226807
(43) Veröffentlichungstag der Anmeldung: 26.10.2016
(73) Patentinhaber: Henkel AG & Co. KGaA, 40589 Düsseldorf (DE)
(72) Erfinder: KNAPPE, Thorsten, 22869 Schenefeld (DE); HEINSOHN, Ulrike, 22529 Hamburg (DE)
(86) Internationale Anmeldenummer: PCT/EP2014/074303
(87) Internationale Veröffentlichungsnummer: WO 2015/090738

(56) Entgegenhaltungen:
- WO-A1-2015/081935
- WO-A1-2015/081937
- WO-A2-2010/130626
- WO-A2-2013/083350
- DE-A1-102008 060 147
- US-A1- 2008 305 064
- GAO TIMOTHY ET AL: "A NEW MULTIFUNCTIONAL, SHINE-ENHANCING EMOLLIENT: PPG-3 BENZYL ETHER MYRISTATE", JOURNAL OF COSMETIC SCIENCE, SOCIETY OF COSMETIC CHEMISTS, NEW YORK, NY, US, Bd. 55, Nr. SUPPL, 1. Januar 2004 (2004-01-01), Seiten S143-S150, XP009077503, ISSN: 1525-7886

## Beschreibung

Die Anmeldung betrifft das technische Fachgebiet der temporären Umformung keratinhaltiger Fasern, insbesondere menschlicher Haare. Gegenstand der Anmeldung sind Aerosolschäume, enthaltend mindestens ein Treibmittel sowie eine kosmetische Zusammensetzung, die mindestens einen festigenden Wirkstoff und mindestens einen speziellen Haarpflegestoff enthält. Gegenstand der Anmeldung ist weiterhin die Verwendung dieser Zusammensetzungen zur temporären Umformung keratinhaltiger Fasern.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als erstrebenswert, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden. Insbesondere die Applikation mittels Aerosolabgabebehälter in Form eines Sprays oder eines Schaums erfreut sich hoher Beliebtheit.

Die wichtigste Eigenschaft eines Mittels zur temporären Verformung keratinischer Fasern, im Folgenden auch Stylingmittel genannt, besteht darin, den behandelten Fasern in der neu modellierten Form - d.h. einer den Fasern aufgeprägten Form - einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge der eingesetzten festigenden Wirkstoffe bestimmt, wobei auch ein Einfluss der weiteren Bestandteile des Stylingmittels sowie der Applikationsform gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums oder des versprühten Aerosols, und Eigenschaften, die die Handhabung des Stylingmittels betreffen, unterteilt werden, wobei den Eigenschaften am Haar besondere Wichtigkeit zukommt. Zu nennen sind neben Feuchtebeständigkeit und/oder niedriger Klebrigkeit insbesondere ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurden im Stand der Technik als festigende Wirkstoffe eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden.

Insbesondere die Applikation in Form eines Aerosols erfordert zusätzliche Maßnahmen. Das Aerosolprodukt muss sich auf der keratinhaltigen Faser gut verteilen lassen, d.h. bei Produkten in Form eines Aerosolschaums muss dieser Schaum voluminös und so hinreichend stabil sein, dass er auf die Faser appliziert werden kann. Gleichzeitig muss dieser Schaum jedoch leicht auf der Faser brechen, um eine ausreichende Benetzung der Fasern mit der Zusammensetzung zu gewährleisten. Eine besonders wichtige Anforderung, die ein modernes Stylingmittel darüber hinaus aufweisen muss, ist ein ausreichender Pflegeeffekt. Darunter wird insbesondere verstanden, dass die Haare nach der Anwendung der Stylingmittel einen weichen Griff und Glanz aufweisen, dass sie sich leicht kämmen lassen, ein hohes Volumen und eine hohe Flexibilität aufweisen. In der Vergangenheit führte die Einarbeitung üblicher Haarkonditionierender Wirkstoffe in Stylingmittel mitunter aber zu einem schlechteren Halt der Haare.

In Dokument WO 2013/083350 wurden Aerosolzusammensetzungen offenbart, die festigende Wirkstoffe und ethoxylierte Verbindungen enthält.

In den Dokumenten DE 102008060147, XP009077503 (ISSN: 1525-7886) und US 2008/305064 werden bestimmte Carbonsäureester für die Verwendung in Haarpflegemitteln, insbesondere zur Verbesserung des Haarglanzes und/oder als Lösungsmittel für Silikonharze, beschrieben.

Aufgabe der vorliegenden Erfindung war es daher, Mittel zur temporären Verformung keratinhaltiger Fasern zur Verfügung zu stellen, die sich durch einen hohen Haltegrad auszeichnen und sich gut auf die keratinhaltigen Fasern applizieren lassen. Die Mittel sollten sich insbesondere als schnell brechender Schaum auf die Faser applizieren lassen. Ferner sollten die Mittel auf den Haaren eine optimale Balance zwischen befriedigendem Halt und gepflegtem Haargefühl hinterlassen. Zur Lösung dieser Aufgabe eignet sich die Kombination eines Treibmittels mit einer kosmetischen Zusammensetzung, die neben Wasser, einen festigenden Wirkstoff und einen speziellen Haarpflegenden Wirkstoff enthält.

Ein erster Gegenstand der vorliegenden Anmeldung ist ein Aerosolschaum, enthaltend
a) mindestens ein Treibmittel und
b) mindestens eine kosmetische Zubereitung, welche
   (i) mindestens einen festigenden Wirkstoff und
   (ii) mindestens einen alkoxylierten Carbonsäureester der nachfolgenden Formel (I) enthält, 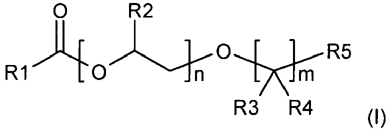 in der
      - R1 für einen geradkettigen oder verzweigten, gesättigten oder ungesättigten Alkylrest mit 6 bis 24 Kohlenstoffatomen steht,
      - R2 für Wasserstoff oder einen Alkylrest mit 1 bis 4 Kohlenstoffatomen steht,
      - R3 oder R4 unabhängig voneinander für Wasserstoff oder einen Alkylrest mit 1 bis 4 Kohlenstoffatomen stehen,
      - R5 für einen geradkettigen oder verzweigten, gesättigten oder ungesättigten Alkylrest mit 6 bis 24 Kohlenstoffatomen oder für einen Phenylrest steht, und
      - n und m unabhängig voneinander für die Zahlen 0 oder 1 bis 20 stehen, und
   (iii) 50 bis 98 Gew.-% Wasser, bezogen auf das Gesamtgewicht des Aerosolsschaums.

Die erfindungsgemäßen Aerosolschäume umfassen a) ein Treibmittel, b) eine kosmetische Zubereitung sowie gegebenenfalls weitere Aktiv- und/oder Hilfsstoffe. Ein erster wesentlicher Bestandteil der erfindungsgemäßen Aerosolschäume ist der in der kosmetischen Zubereitung enthaltene festigende Wirkstoff. Ein festigender Wirkstoff im Sinne der Erfindung trägt im Rahmen der temporären Umformung keratinhaltiger Fasern zum Halt der aufgeprägten Form der Fasern (bei Haaren insbesondere der Halt einer Frisur oder des Haarvolumens) bei. Als eine Testmethode für die festigende Wirkung eines Wirkstoffs wird häufig der so genannte curl-retention-Test angewendet.

Bevorzugte erfindungsgemäße Aerosolschäume sind dadurch gekennzeichnet, dass der Gewichtsanteil des festigenden Wirkstoffs (i) am Gesamtgewicht der Aerosolzusammensetzung 0,5 bis 12 Gew.-%, vorzugsweise 1,0 bis 10 Gew.-% und insbesondere 2,0 bis 8,0 Gew.-% beträgt.

Als festigende Wirkstoffe eignen sich beispielsweise festigende Polymere oder Wachse. Im Rahmen einer Ausführungsform der Erfindung ist es bevorzugt, dass der festigende Wirkstoff ausgewählt wird aus mindestens einem festigenden Polymer aus der Gruppe, die gebildet wird aus festigenden nichtionischen Polymeren, festigenden anionischen Polymeren, festigenden amphoteren Polymeren und festigenden kationischen Polymeren. Bevorzugte erfindungsgemäße Aerosolschäume enthalten mindestens ein festigendes kationisches Polymer. Die festigenden kationischen Polymere weisen mindestens eine Struktureinheit auf, die mindestens ein permanent kationisiertes Stickstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammoniumverbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Bevorzugte Aerosolschäume enthalten das festigende kationische Polymer in einer Menge von 0,05 Gew.-% bis 10 Gew.-%, besonders bevorzugt von 0,075 Gew.-% bis 8,0 Gew.-%, ganz besonders bevorzugt von 0,1 Gew.-% bis 5,0 Gew.-%, jeweils bezogen auf das Gewicht der besagten Zubereitung. Die kationischen festigenden Polymere können erfindungsgemäß aus kationischen, quaternisierten Cellulose-Derivaten ausgewählt werden. Als vorteilhaft haben sich kationische, quaternisierte Cellulosen erwiesen, die in einer Seitenkette mehr als eine permanente kationische Ladung tragen. Besonders bevorzugt sind dabei kationische Cellulosederivate, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat® H 100 und Celquat® L 200 von der Firma National Starch vertrieben werden. Bevorzugte Aerosolzusammensetzungen sind dadurch gekennzeichnet, dass die Zubereitung a) als festigenden Wirkstoff mindestens ein kationisches Cellulosederivat enthält.

Weiterhin eignen sich solche kationischen festigenden Polymere, die mindestens eine Struktureinheit der Formel (M-I) und mindestens eine Struktureinheit der Formel (M-II) und gegebenenfalls mindestens eine Struktureinheit der Formel (M-III) umfassen 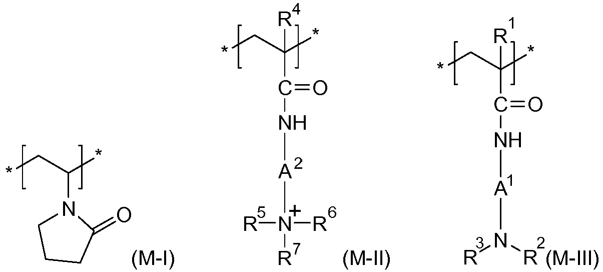 worin
R¹ und R⁴ unabhängig voneinander für ein Wasserstoffatom oder eine Methylgruppe stehen,
A¹ und A² unabhängig voneinander für eine Gruppe Ethan-1,2-diyl, Propan-1,3-diyl oder Butan-1,4-diyl stehen,
R², R³, R⁵ und R⁶ unabhängig voneinander für eine (C₁ bis C₄)-Alkylgruppe stehen,
R⁷ für eine (C₈ bis C₃₀)-Alkylgruppe steht.

Zur Kompensation der positiven Ladung dieses wie aller weiteren kationischen Polymere können alle physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat, p-Toluolsulfonat oder Triflat eingesetzt werden.

Geeignete kationische Polymere sind beispielsweise als
- Copolymere aus mit Diethylsulfat quaterniertem Dimethylaminoethylmethacrylat, mit N-Vinylpyrrolidon mit der INCI-Bezeichnung Polyquaternium-11 unter den Bezeichnungen Gafquat® 440, Gafquat®734, Gafquat®755 (jeweils Firma ISP) sowie Luviquat PQ 11 PN (Firma BASF SE),
- Copolymere aus Methacryloylaminopropyllauryldimethylammoniumchlorid mit N-Vinylpyrrolidon und Dimethylaminopropylmethacrylamid mit der INCI-Bezeichnung Polyquaternium-55 unter den Handelsnamen Styleze® W-10, Styleze® W-20 (Firma ISP),
- Copolymere aus Methacryloylaminopropyllauryldimethylammoniumchlorid mit N-Vinylpyrrolidon, N-Vinylcaprolactam und Dimethylaminopropylmethacrylamid mit der INCI-Bezeichnung Polyquaternium-69 unter den Handelsnamen Aquastyle® 300 (Firma ISP),
im Handel erhältlich.

Weitere bevorzugte kationische festigende Copolymere enthalten mindestens ein Strukturelement der Formel (M-IV) 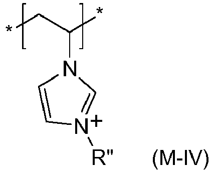 worin
R" für eine (C₁ bis C₄)-Alkylgruppe, insbesondere eine Methylgruppe, steht,
und zusätzlich mindestens ein weiteres kationisches und/oder nichtionisches Strukturelement.

Es ist wiederum im Rahmen dieser Ausführungsform erfindungsgemäß bevorzugt, wenn in der erfindungsgemäßen Zubereitung als kationisches festigendes Polymer mindestens ein kationisches Copolymer enthalten ist, das neben mindestens einem Strukturelement der Formel (M-IV) zusätzlich ein Strukturelement der Formel (M-I) umfasst 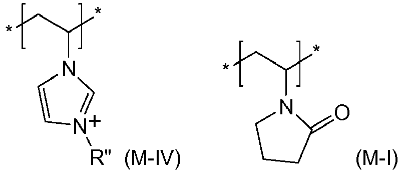 worin
R" für eine (C₁ bis C₄)-Alkylgruppe, insbesondere eine Methylgruppe, steht. Ganz besonders bevorzugte kationische festigende Polymere enthalten 10 bis 30 Mol-%, vorzugsweise 15 bis 25 Mol.-% und insbesondere 20 Mol.-% Struktureinheiten gemäß Formel (M-IV) und 70 bis 90 Mol.-%, vorzugsweise 75 bis 85 Mol.-% und insbesondere 80 Mol.-% Struktureinheiten der Formel (M-I).

Hierbei ist es besonders bevorzugt, wenn die kationischen Polymere neben Polymereinheiten, die aus dem Einbau der genannten Struktureinheiten der Formeln (M-IV) und (M-I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M-IV) mit R" = Methyl und (M-I) aufgebaut und lassen sich durch die allgemeine Formel (Poly1) 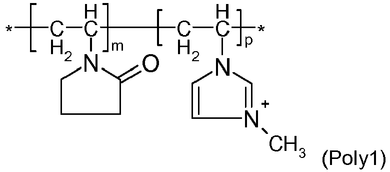 beschreiben, wobei die Indices m und p je nach Molmasse des Polymers variieren und nicht bedeuten sollen, dass es sich um Blockcopolymere handelt. Vielmehr können Struktureinheiten der Formel (M-IV) und der Formel (M-I) im Molekül statistisch verteilt vorliegen.

Wird zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat® Style, Luviquat® FC 370, Luviquat® FC 550, Luviquat® FC 905 und Luviquat® HM 552 erhältlich.

Wird zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der Firma BASF unter den Handelsnamen Luviquat® UltraCare erhältlich.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (M-V) aufweisen

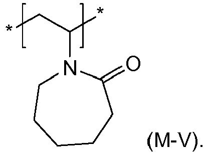

Weitere besonders bevorzugte erfindungsgemäße Zubereitungen dieser Ausführungsform sind somit dadurch gekennzeichnet, dass sie als kationisches festigendes Polymer mindestens ein Copolymer (c2) enthalten, das mindestens eine Struktureinheit der Formel (M-IV-a) und mindestens Struktureinheit der Formel (M-I) und mindestens Struktureinheit der Formel (M-V) enthält

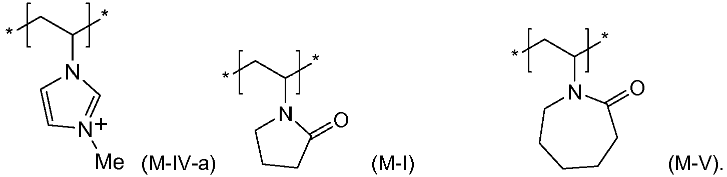

Auch hierbei ist es im Rahmen dieser Ausführungsform besonders bevorzugt, wenn die Copolymere (c2) neben Polymereinheiten, die aus dem Einbau der genannten Struktureinheiten der Formeln (M-IV-a), (M-I) und (M-V) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M-IV-a), (M-I) und (M-V) aufgebaut und lassen sich durch die allgemeine Formel (Poly2) 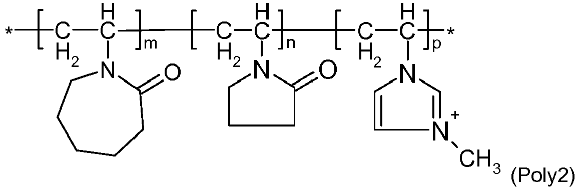 beschreiben, wobei die Indices m, n und p je nach Molmasse des Polymers variieren und nicht bedeuten sollen, dass es sich um Blockcopolymere handelt. Vielmehr können Struktureinheiten der besagten Formeln im Molekül statistisch verteilt vorliegen.

Wird zur Kompensation der positiven Ladung des Polymer der Formel (Poly2) ein Methosulfat verwendet, werden solche N-Methylvinylimidazol/Vinylpyrrolidon/Vinylcaprolactam-Copolymere laut INCI-Nomenklatur als Polyquarternium-46 bezeichnet und sind beispielsweise von der Firma BASF unter dem Handelsnamen Luviquat® Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten der Formel (M-IV-a) und 30 bis 50 Mol.-%, vorzugsweise 35 bis 45 Mol.-% und insbesondere 40 Mol.-% Struktureinheiten der Formel (M-I) und 40 bis 60 Mol.-%, vorzugsweise 45 bis 55 Mol.-% und insbesondere 60 Mol.-% Struktureinheiten der Formel (M-V).

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M-IV-a) und (M-I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Zubereitungen dieser Ausführungsform sind dadurch gekennzeichnet, dass sie als kationisches festigendes Polymer mindestens ein Copolymer (c3) enthalten, das mindestens eine Struktureinheit der Formel (M-IV-a), mindestens eine weitere Struktureinheit der Formel (M-I), mindestens eine weitere Struktureinheit der Formel (M-VI) und mindestens eine weitere Struktureinheit gemäß Formel (M-VII) enthält

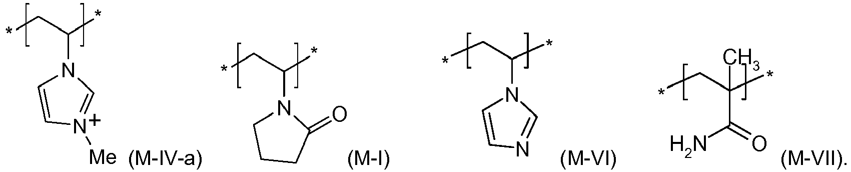

Auch hierbei ist es im Rahmen dieser Ausführungsform besonders bevorzugt, wenn die Copolymere (c3) neben Polymereinheiten, die aus dem Einbau der genannten Struktureinheiten der Formeln (M-IV-a), (M-I), (M-VI) und (M-VII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formeln (M-IV-a), (M-I), (M-VI) und (M-VII) aufgebaut und lassen sich durch die allgemeine Formel (Poly3) 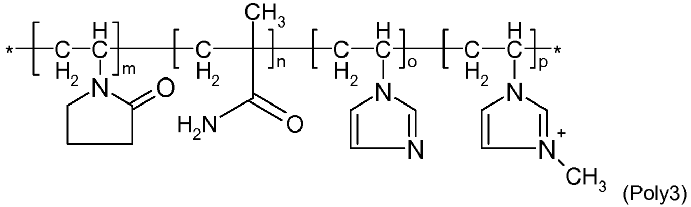 beschreiben, wobei die Indices m, n, o und p je nach Molmasse des Polymers variieren und nicht bedeuten sollen, dass es sich um Blockcopolymere handelt. Vielmehr können Struktureinheiten der Formeln (M-IV-a), (M-I), (M-VI) und (M-VII) im Molekül statistisch verteilt vorliegen.

Wird zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der Firma BASF unter dem Handelsnamen Luviquat® Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten der Formel (M-IV-a) und 45 bis 65 Mol.-%, vorzugsweise 50 bis 60 Mol.-% und insbesondere 55 Mol.-% Struktureinheiten der Formel (M-I) und 1 bis 20 Mol.-%, vorzugsweise 5 bis 15 Mol.-% und insbesondere 10 Mol.-% Struktureinheiten der Formel (M-VI) und 20 bis 40 Mol.-%, vorzugsweise 25 bis 35 Mol.-% und insbesondere 29 Mol.-% Struktureinheiten der Formel (M-VII).

Unter den zusätzlichen festigenden Polymer ausgewählt aus den kationischen Polymeren mit mindesten einem Strukturelement der obigen Formel (M-IV), gelten als bevorzugt:
- N-Vinylpyrrolidon/1-Vinyl-3-methyl-1H-imidazoliumchlorid-Copolymere (wie beispielsweise Polymere mit der INCI-Bezeichnung Polyquaternium-16 unter den Handelsbezeichnungen Luviquat® Style, Luviquat® FC 370, Luviquat® FC 550, Luviquat® FC 905 und Luviquat® HM 552 (BASF SE)),
- N-Vinylpyrrolidon/1-Vinyl-3-methyl-1H-imidazoliummethylsulfat-Copolymere (wie beispielsweise Polymere mit der INCI-Bezeichnung Polyquaternium-44 unter der Handelsbezeichnung Luviquat® Care (BASF SE)),
- N-Vinylpyrrolidon/N-Vinylcaprolactam/1-Vinyl-3-methyl-1H-imidazolium-Terpolymer (wie beispielsweise Polymere mit der INCI-Bezeichnung Polyquaternium-46 unter den Handelsbezeichnungen Luviquat® Care oder Luviquat® Hold (BASF SE)),
- N-Vinylpyrrolidon/Methacrylamid/N-Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat® Supreme (BASF SE)),
sowie Gemische aus diesen Polymeren.

Bevorzugte erfindungsgemäße Zusammensetzungen enthalten mindestens ein festigendes nichtionisches Polymer. Dieses festigende nichtionische Polymer kann in den Zusammensetzungen als alleiniges Polymer enthalten sein. Mit besonderem Vorzug wird das mindestens eine festigende nichtionische Polymer jedoch in Kombination mit mindestens einem festigenden kationischen Polymer eingesetzt. Im Rahmen einer besonders bevorzugten Ausführungsform enthalten die Zubereitungen b) mindestens ein festigendes nichtionisches Polymer und mindestens ein festigendes kationisches Polymer.

Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die festigenden nichtionischen Polymere sind in der erfindungsgemäßen Zubereitung dieser Ausführungsform bevorzugt in einer Menge von 0,1 Gew.-% bis 12,0 Gew.-%, besonders bevorzugt von 0,2 Gew.-% bis 10,0 Gew.-%, ganz besonders bevorzugt von 0,5 Gew.-% bis 8,0 Gew.-%, jeweils bezogen auf das Gewicht der besagten Zubereitung, enthalten.

Es sind erfindungsgemäß solche festigende nichtionische Polymere mit mindestens einem Strukturelement der Formel (M-VIII) 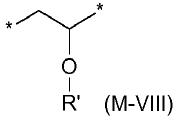 bevorzugt geeignet, welche gemäß der Formel (M-VIII) als R' ein Wasserstoffatom, eine Acetylgruppe oder eine Propanoylgruppe, insbesondere eine Acetylgruppe, tragen.

Die festigenden nichtionischen Polymere werden wiederum bevorzugt ausgewählt aus mindestens einem Polymer der Gruppe, die gebildet wird aus
- Homopolymeren und nichtionischen Copolymeren des N-Vinylpyrrolidons,
- nichtionischen Copolymeren des Isobutens.

Geeignete Polyvinylpyrrolidone sind beispielsweise Handelsprodukte wie Luviskol® K 90 oder Luviskol® K 85 der Firma BASF SE.
Geeignete Polyvinylalkohole werden beispielsweise unter den Handelsbezeichnungen Elvanol® von Du Pont oder Vinol® 523/540 von der Firma Air Products vertrieben.
Geeignetes Polyvinylacetat wird beispielsweise unter dem Handelsnamen Vinac® als Emulsion von der Firma Air Products vertrieben.

Mittel, die als festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid, sind erfindungsgemäß ganz besonders bevorzugt.

Weitere mögliche erfindungsgemäße Zubereitungen der Ausführungsform mit zusätzlichem nichtionischen festigendem Polymer sind dadurch gekennzeichnet, dass sie als nichtionisches festigendes Polymer mindestens ein Copolymer enthalten, das mindestens eine weitere Struktureinheit der Formel (M-I), mindestens eine weitere Struktureinheit der Formel (M-VI) und mindestens eine weitere Struktureinheit der Formel (M-VII) enthält

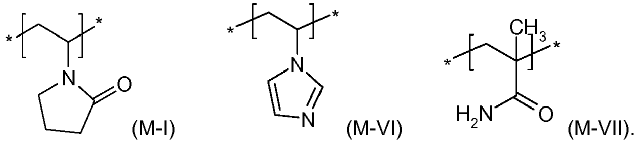

Auch hierbei ist es besonders bevorzugt, wenn diese Copolymere neben Polymereinheiten, die aus dem Einbau der genannten Struktureinheiten gemäß der Formeln (M-IV-a), (M-I), (M-VI) und (M-VII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c4) ausschließlich aus Struktureinheiten der Formeln (M-IV-a), (M-I), (M-VI) und (M-VII) aufgebaut und lassen sich durch die allgemeine Formel (Poly4) 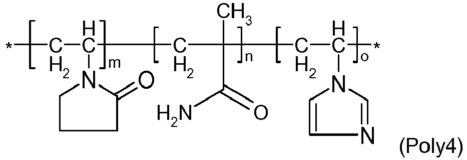 beschreiben, wobei die Indices m, n, o und p je nach Molmasse des Polymers variieren und nicht bedeuten sollen, dass es sich um Blockcopolymere handelt. Vielmehr können Struktureinheiten der Formeln (M-I), (M-VI) und (M-VII) im Molekül statistisch verteilt vorliegen.

Ein besonders bevorzugtes Polymer wird dabei ausgewählt aus den Polymeren mit der INCI-Bezeichnung VP/Methacrylamide/Vinyl Imidazole Copolymer, die beispielsweise unter dem Handelsnamen Luviset Clear von der Fa. BASF SE erhältlich sind.

Wie den vorherigen Ausführungen zu den kationischen und den nichtionischen festigenden Polymeren zu entnehmen ist, sind erfindungsgemäß solche Aerosolzusammensetzungen besonders bevorzugt, bei denen die Zubereitung b) als festigenden Wirkstoff (i) mindestens ein Vinylpyrrolidon Copolymer enthält.

Bevorzugt sind weiterhin Aerosolschäume, in denen die Zubereitung b) als festigenden Wirkstoff (i) mindestens ein nichtionisches festigendes Polymer und mindestens ein kationisches festigendes Polymer enthält.

Ganz besonders bevorzugte Aerosolschäume sind dadurch gekennzeichnet, dass die Zubereitung b) als festigenden Wirkstoff (i) mindestens ein nichtionisches festigendes Polymer aus der Gruppe der Vinylpyrrolidon Copolymere und mindestens ein kationisches festigendes Polymer aus der Gruppe der Vinylpyrrolidon Copolymere enthält.

Die erfindungsgemäßen Zubereitungen können als festigendes Polymer auch mindestens ein festigendes amphoteres Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefasst, die -COOH- oder SO₃H-Gruppen und quartäre Ammoniumgruppen enthalten. Ein Beispiel für ein erfindungsgemäß einsetzbares festigendes amphoteres Polymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe der Acrylsäure, Methacrylsäure und deren (C₁ bis C₄)-Alkylestern darstellt.

Letztere weisen zusätzlich zu der kationogenen Gruppe bzw. positiv geladenen Gruppe mindestens eine negativ geladene Gruppe im Molekül auf und werden auch als zwitterionische Polymere bezeichnet.

Die festigenden amphoteren Polymere sind in den erfindungsgemäßen Zubereitungen bevorzugt in Mengen von 0,1 Gew.-% bis 20 Gew.-%, besonders bevorzugt von 0,05 bis 10 Gew.-%, jeweils bezogen auf das Gewicht der besagten Zubereitung, enthalten. Mengen von 0,1 bis 5 Gew.-% sind ganz besonders bevorzugt.

Weiterhin können als festigende Polymere mindestens ein filmbildendes anionisches Polymer eingesetzt werden. Bei anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte festigende anionische Polymere sind Acrylsäure-Acrylamid-Copolymere sowie insbesondere Polyacrylamidcopolymere mit Sulfonsäuregruppen-haltigen Monomeren. Ein besonders bevorzugtes festigendes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Handelsprodukts, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.
Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte festigende anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Weitere bevorzugt einsetzbare festigende anionische Polymere werden ausgewählt aus mindestens einem Polymer der Gruppe, die gebildet wird aus
- Copolymeren aus Vinylacetat und Crotonsäure (wie sie beispielsweise als Handelsprodukt Aristoflex® A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex® Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie sie unter dem Handelsnamen Luviset PUR mit der INCI-Bezeichnung Polyurethane-1 oder unter der Handelsbezeichnung Luviset Shape mit der INCI-Bezeichnung Polyurethane-34 von der Firma BASF SE vertrieben werden).

Alternativ oder in Kombination mit den festigenden Polymeren enthalten bevorzugte Zubereitungen b) als festigenden Wirkstoff mindestens ein Wachs. Aerosolschäume umfassend eine Zubereitung b), die als festigenden Wirkstoff mindestens ein Wachs enthält, werden erfindungsgemäß bevorzugt. Im Rahmen der Erfindung eingesetzte Wachse sind bei 20°C knetbar, fest bis brüchig hart, grob bis feinkristallin, durchscheinend bis opak, jedoch nicht glasartig; über 40 °C ohne Zersetzung schmelzend. Wachse unterscheiden sich von ähnlichen synthetischen oder natürlichen Produkten (z. B. Harzen, plastischen Massen, Metallseifen usw.) darin, dass sie von 40°C bis 90 °C in den schmelzflüssigen, niedrigviskosen Zustand übergehen.

Es sind erfindungsgemäß Wachse bevorzugt, die bei 1013 mbar einen Schmelzpunkt im Bereich von 50 °C bis 85 °C, insbesondere von 60°C bis 75 °C, aufweisen.

Bevorzugt werden die Wachse aus pflanzlichen, tierischen und mineralischen Wachsen ausgewählt. Erfindungsgemäß einsetzbare Wachse sind natürliche Wachse (pflanzliche Wachse: Baumwollwachs, Carnaubawachs, Candelillawachs, Espartowachs, Guarumawachs, Japanwachs, Korkwachs, Montanwachs, Ouricurywachs, Reiskeimölwachs, Zuckerrohrwachs; tierische Wachse: Bienenwachs, Bürzeldrüsenfett, Wollwachs, Schellackwachs (siehe Schellack), Walrat; Mineralwachse: Mikrowachse, Ceresin, Ozokerit), chemisch modifizierte Wachse (Hartwachse: hydrierte Jojobawachse (siehe Jojobaöl), Montanwachs, Sasolwachse) und synthetische Wachse (Polyalkylenwachse (wie Polyolefinwachse, Polyethylenwachse, Polypropylenwachse), Polyethylenglycolwachse, Amidwachse). Erfindungsgemäß besonders bevorzugte Wachse sind Bienenwachs (Cera Alba), Carnaubawachs, Candelillawachs, Montanwachs, mikrokristalline Wachse (mikrokristalline Paraffine) und Cetylpalmitat.

Die erfindungsgemäße Lehre umfasst auch den kombinierten Einsatz von mehreren Wachsen in den erfindungsgemäßen Zubereitungen. So kann ein Zusatz geringer Mengen an Carnaubawachs z.B. dazu verwendet werden, um den Schmelz- und Tropfpunkt eines anderen Wachses zu erhöhen. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax® GP 200 (eine Mischung von Stearylalkohol und Polyethylenglykolstearat mit einem Schmelzpunkt von 47-51 °C; Hersteller: Croda) und "Weichceresin® FL 400" (ein VaselineA/aselinöl/Wachs-Gemisch mit einem Schmelzpunkt von 50-54 °C; Hersteller:
Parafluid Mineralölgesellschaft) erhältlichen Wachsmischungen sind Beispiele für erfindungsgemäß bevorzugt eingesetzte Mischungen.

Die erfindungsgemäßen Aerosolschäume enthalten die Wachse bezogen auf ihr Gesamtgewicht vorzugsweise in Mengen von 0,1 bis 10 Gew.-% und insbesondere von 0,2 bis 5,0 Gew.-%.

In erfindungsgemäß bevorzugten Aerosolschäumen beträgt der Gewichtsanteil des festigenden Wirkstoffs am Gesamtgewicht der Aerosolzusammensetzung 0,5 bis 12 Gew.-%, vorzugsweise 1,0 bis 10 Gew.-% und insbesondere 2,0 bis 8,0 Gew.-%.

Ein weiterer wesentlicher Bestandteil der Zubereitung b) ist ein alkoxylierter Carbonsäureester der zuvor genannten Formel (I). Die Inkorporation mindestens eines Esters nach Formel (I) in die erfindungsgemäßen Aerosolschäume führt dazu, dass diese auf den damit behandelten Haaren einen sehr guten Pflegeeffekt hervorrufen, ohne den Halt der Haare in negativer Weise zu beeinflussen.

In bevorzugten Estern nach Formel (I) steht der Rest R1 für einen geradkettigen, gesättigten Alkylrest, der vorzugsweise 8 bis 18 Kohlenstoffatomen aufweist. Besonders bevorzugt steht der Rest R1 für einen Decyl-, Lauryl-, Myristyl-, Palmityl- oder Stearylrest und insbesondere bevorzugt für einen Myristylrest. In weiterhin bevorzugten Estern nach Formal (I) steht der Rest R2 für einen Methyl- oder einen Ethylrest, vorzugsweise für einen Methylrest, und die Reste R3 und R4 stehen für gleiche Reste, vorzugsweise für -H oder für einen Methylrest und insbesondere bevorzugt für Wasserstoff.
In weiterhin bevorzugten Estern gemäß Formel (I) hat der Rest R5 die gleiche Bedeutung wie der Rest R1 oder der Rest R5 steht für eine - gegebenenfalls durch eine oder mehrere Hydroxylgruppen, Aminogruppen, Methyl-, Ethyl-, Methoxy-, oder Ethoxygruppen substituierte Phenylgruppe. Vorzugsweise steht der Rest R5 für eine nicht substituierte Phenylgruppe.
Die Indices n und m stehen unabhängig voneinander bevorzugt für die Zahl 0 oder für eine Zahl von 1 bis 10. Besonders bevorzugt steht n für eine Zahl von 1 bis 10, bevorzugt von 2 bis 7 und insbesondere von 3 bis 5, und m steht für die Zahlen 1, 2 oder 3, bevorzugt für 1 oder 2 und insbesondere für die Zahl 1.

Ein besonders geeigneter Carbonsäureester gemäß Formel (I) ist dadurch gekennzeichnet, dass der Rest R1 für einen Myristylrest, R2 für einen Methylrest, die Reste R3 und R4 für Wasserstoff, R5 für einen unsubstituierten Phenylrest, n für die Zahl 3 und M für die Zahl 1 steht.

Solche Ester sind bekannt unter der INCI-Bezeichnung PPG-3 Benzyl Ether Myristate und im Handel von verschiedenen Anbietern erhältlich, beispielsweise von der Firma Croda unter der Handelsbezeichnung Crodamol STS®.

In einer besonders bevorzugten Ausführungsform sind erfindungsgemäße Aerosolschäume dadurch gekennzeichnet, dass die Zubereitung b) einen alkoxylierten Carbonsäureester gemäß Formel (I) enthält, in der
- R1 für einen geradkettigen, gesättigten Alkylrest mit 8 bis 18 Kohlenstoffatomen, bevorzugt für einen Decyl-, Lauryl-, Myristyl-, Palmityl- oder Stearylrest,
- R2 für einen Methylrest,
- R3 und R4 jeweils für Wasserstoff,
- R5 für einen Phenylrest,
- n für eine Zahl von 1 bis 10, bevorzugt von 2 bis 7, und
- m für die Zahlen 1 oder 2 steht.

Der Gewichtsanteil des mindestens einen Carbonsäureesters gemäß Formel (I) am Gesamtgewicht der Aerosolschäume beträgt bevorzugt 0,1 bis 5,0 Gew.-%, vorzugsweise 0,2 bis 3,0 Gew.-% und insbesondere 0,3 bis 2,0 Gew.-%.

Als weiteren Bestandteil neben der kosmetischen Zubereitung b) umfassen die erfindungsgemäßen Aerosolschäume mindestens ein Treibmittel a). Bevorzugte Treibmittel a) enthalten mindestens ein Treibmittel aus der Gruppe von Propan und Butan. Besonders bevorzugt ist eine Mischung von Propan und Butan in einem Gewichtsverhältnis von Propan zu Butan von 5 : 1 bis 1 : 5, bevorzugt von 4 : 1 bis 1 : 4 und insbesondere von 3 : 1 bis 1 : 3.

Besonders bevorzugte Aerosolschäume sind dadurch gekennzeichnet, dass das Treibmittel a) ausgewählt ist aus Propan und/oder Butan, wobei der gemeinsame Gewichtsanteil von Propan und/oder Butan am Gesamtgewicht der Aerosolzusammensetzung 1-99,5 Gew.-%, vorzugsweise 4 bis 50 Gew.-% beträgt.

Die erfindungsgemäßen Aerosolschäume werden vorzugsweise in einem Druckbehälter konfektioniert. Ein "Druckbehälter" ist erfindungsgemäß ein Behälter, der im Inneren einen höheren Gasdruck aufweist als außerhalb des Behälters und aus dem sich ein Gasstrom über ein Ventil entnehmen lässt. Drückbehälter, mit deren Hilfe ein Produkt (z.B. eine flüssige Zusammensetzung) durch den inneren Gasdruck des Behälters über ein Ventil abgegeben wird, bezeichnet man definitionsgemäß als "Aerosolabgabebehälter". Besonders bevorzugt ist die Konfektionierung der Aerosolzusammensetzung in einem Aerosolabgabebehälter.
Als "Nichtaerosolabgabebehälter" wird im Umkehrschluss zur Aerosoldefinition ein Behältnis unter Normaldruck definiert, mit dessen Hilfe ein Produkt mittels mechanischer Einwirkung durch ein Pump- oder Quetschsystem abgegeben werden kann.

Die erfindungsgemäßen Aerosolzusammensetzungen lassen sich in üblicher Art und Weise herstellen. In der Regel werden alle Bestandteile der Zubereitung der erfindungsgemäßen Aerosolschäume in einen geeigneten druckfesten Behälter eingefüllt. Dieser wird daraufhin mit einem Ventil verschlossen. Über herkömmliche Techniken wird schließlich die gewünschte Menge des speziellen Treibmittels eingefüllt.

Als druckfeste Behälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber dem im Druckbehälter konfektionisierten Mittel. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein.
Die erfindungsgemäßen Zusammensetzungen können auch in einem Mehrkammerspender verpackt sein. Der Mehrkammerspender kann auch so eingesetzt werden, dass eine Kammer mit dem komprimierten Treibmittel und eine andere Kammer mit den restlichen Bestandteilen der erfindungsgemäßen Aerosolzusammensetzung befüllt ist. Ein derartiger Mehrkammerspender ist beispielsweise eine so genannte Bag-in-Can-Verpackung.
Die erfindungsgemäßen kosmetischen Zubereitungen b) enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Bevorzugte Aerosolschäume sind dadurch gekennzeichnet, dass sie bezogen auf ihr Gesamtgewicht-60 bis 95 Gew.-% und insbesondere 70 bis 90 Gew.-% Wasser enthalten.

Durch den Zusatz von Propylenglykol und/oder Polyethylenglykol und/oder Polypropylenglykol kann die Flexibilität des bei der Anwendung der erfindungsgemäßen Aerosolschäume gebildeten Halts erhöht werden. Wird also ein flexiblerer Halt gewünscht, enthalten die erfindungsgemäßen Aerosolschäume bezogen auf ihr Gesamtgewicht vorzugsweise 0,01 bis 30 Gew.-% Polyethylenglykol und/oder Polypropylenglykol.

Die kosmetischen Zubereitungen b) weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Effekte lassen sich durch Zusatz mindestens eines (C₂ bis C₆)-Trialkylcitrats zur erfindungsgemäßen Zubereitung b) steigern. Daher ist es erfindungsgemäß bevorzugt, wenn die Zubereitung b) zusätzlich mindestens eine Verbindung der Formel (E) enthält, 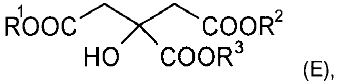 worin
R¹, R² und R³ unabhängig voneinander für eine (C₂ bis C₆)-Alkylgruppe stehen.

Beispiele einer (C₂ bis C₆)-Alkylgruppe gemäß der Formel (E) sind Methyl, Ethyl, Isopropyl, n-Propyl, n-Butyl, sec-Butyl, Isobutyl, tert.-Butyl, n-Pentyl, Neopentyl, Isopentyl, n-Hexyl. Als besonders wirkungsvolle Verbindung der Formel (E) stellte sich Triethylcitrat heraus. Die erfindungsgemäße kosmetische Zubereitung b) enthält die Verbindungen der Formel (E) bevorzugt in einer Menge von 0,01 bis 1 Gew.-%, insbesondere von 0,05 bis 0,3 Gew.-%, jeweils bezogen auf das Gewicht der besagten Zubereitung.

Bevorzugt enthalten die erfindungsgemäßen Zubereitungen b) vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Der Einsatz mindestens eines nichtionischen Tensids und/oder mindestens eines kationischen Tensids ist im Rahmen dieser Ausführungsform der Erfindung bevorzugt.
Die zusätzlichen Tenside sind in der erfindungsgemäßen Zubereitung bevorzugt in einer Menge von 0,01 Gew.-% bis 5 Gew.-%, besonders bevorzugt von 0,05 Gew.-% bis 0,5 Gew.-%, jeweils bezogen auf das Gewicht der besagten Zubereitung, enthalten.

Es hat sich als besonders bevorzugt erwiesen, wenn die erfindungsgemäßen kosmetischen Zubereitungen b) zusätzlich mindestens ein kationisches Tensid enthalten. Die pflegenden Eigenschaften der erfindungsgemäßen Aerosolschäume konnten durch die Zugabe des kationischen Tensids zu der Zubereitung b) noch weiter gesteigert werden, ohne dass der Halt der Frisur dadurch negativ beeinträchtigt wurde.

Besonders geeignete kationische Tenside sind bevorzugt ausgewählt aus Tensiden vom Typ der quartären Ammoniumverbindungen, der Esterquats und/oder der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.
Besonders bevorzugt sind Alkyltrimethylammoniumchloride, Dialkyldimethylammoniumchloride und Trialkylmethylammoniumchloride, in denen die langen Alkylketten bevorzugt 10 bis 18 Kohlenstoffatome aufweisen. Insbesondere bevorzugt sind Cetyltrimethylammoniumchlorid und/oder Stearyltrimethylammoniumchlorid.
Das oder die kationischen Tensid(e) wird(werden) in den erfindungsgemäßen Aerosolschäume bevorzugt in einem Gewichtsanteil von 0,02 bis 5,0 Gew.-%, bevorzugt von 0,05 bis 3 Gew.-% und insbesondere von 0,1 bis 2 Gew.-% bezogen auf das Gesamtgewicht der Aerosolzusammensetzungen eingesetzt.

Es hat sich weiterhin als besonders bevorzugt erwiesen, wenn die erfindungsgemäßen kosmetischen Zubereitungen b) zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe,
- mit einem Methyl- oder C₂ - C₆ -Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 Kohlenstoffatomen, an Fettsäuren mit 8 bis 30 Kohlenstoffatomen und an Alkylphenole mit 8 bis 15 Kohlenstoffatomen in der Alkylgruppe, wie beispielsweise die unter den Bezeichnungen Dehydol® LS, Dehydol® LT (Cognis) erhältlichen Produkte,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (T-I)

   R¹CO-(OCH₂CHR²)_{w}OR³ (T-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (T-II),

   R⁴O-[G]Pₚ (T-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (T-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem Cs-Cis-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁴ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.
Ganz besonders bevorzugt enthalten die erfindungsgemäßen Zubereitungen als Tensid mindestens ein Anlagerungsprodukt von 15 bis 100 Mol Ethylenoxid, insbesondere von 15 bis 50 Mol Ethylenoxid an einen linearen oder verzweigten (insbesondere linearen) Fettalkohol mit 8 bis 22 Kohlenstoffatomen und/oder an Rizinusöl. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden, oder um PEG-40 Hydrogenated Castor Oil, das als Eumulgin® CO 455 vermarktet wird.

Bevorzugte Aerosolschäume sind dadurch gekennzeichnet, dass die Zubereitung b) mindestens ein nichtionisches Tensid, vorzugsweise ein nichtionisches Tensid aus der Gruppe der alkoxylierten Fettalkohole und/oder des alkoxylierten Rizinusöls, enthält.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 Kohlenstoffatomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammoniumsowie der Mono-, Di- und Trialkanolammoniumsalze mit 2 bis 4 Kohlenstoffatomen in der Alkanolgruppe,
- lineare und verzweigte Fettsäuren mit 8 bis 30 Kohlenstoffatomen (Seifen),
- Ethercarbonsäuren der Formel R-O-(CH₂-CH₂O)x-CH₂-COOH, in der R eine lineare Alkylgruppe mit 8 bis 30 Kohlenstoffatomen und x = 0 oder 1 bis 16 ist,
- Acylsarcoside mit 8 bis 24 Kohlenstoffatomen in der Acylgruppe,
- Acyltauride mit 8 bis 24 Kohlenstoffatomen in der Acylgruppe,
- Acylisethionate mit 8 bis 24 Kohlenstoffatomen in der Acylgruppe,
- Sulfobernsteinsäuremono- und -dialkylester mit 8 bis 24 Kohlenstoffatomen in der Alkylgruppe und Sulfobernsteinsäuremono-alkylpolyoxyethylester mit 8 bis 24 Kohlenstoffatomen in der Alkylgruppe und 1 bis 6 Oxyethylgruppen,
- lineare Alkansulfonate mit 8 bis 24 Kohlenstoffatomen,
- lineare Alpha-Olefinsulfonate mit 8 bis 24 Kohlenstoffatomen,
- Alpha-Sulfofettsäuremethylester von Fettsäuren mit 8 bis 30 Kohlenstoffatomen,
- Alkylsulfate und Alkylpolyglykolethersulfate der Formel R-O(CH₂-CH₂O)x-OSO₃H, in der R eine bevorzugt lineare Alkylgruppe mit 8 bis 30 Kohlenstoffatomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 Kohlenstoffatomen und 1 bis 6 Doppelbindungen,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 Kohlenstoffatomen darstellen,
- Alkyl- und/oder Alkenyletherphosphate der Formel (T-V), 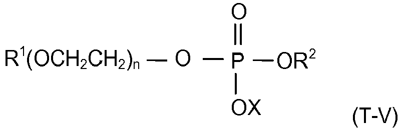 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (T-VI)

   R⁷CO(AlkO)ₙSO₃M (T-VI)

   in der R⁷CO- für einen linearen oder verzweigten, aliphatischen, gesättigten und/oder ungesättigten Acylrest mit 6 bis 22 Kohlenstoffatomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (T-VII) 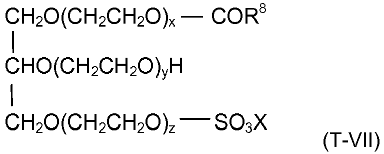 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (T-VII) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon®- Typen, Gluadin®- Typen, Hostapon® KCG oder die Amisoft®- Typen.

Als zwitterionische Tenside werden solche oberflächenaktiven Verbindungen bezeichnet, die im Molekül mindestens eine quartäre Ammoniumgruppe und mindestens eine -COO(-) - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 Kohlenstoffatomen in der Alkylgruppe. Besonders bevorzugte Ampholyte sind das N-Kokosalkylaminopropionat, das Kokosacylaminoethyl-aminopropionat und das C₁₂ - C₁₈ - Acylsarcosin.

Im Rahmen einer erfindungsgemäß bevorzugten Ausführungsform enthalten die kosmetischen Zubereitungen b) zusätzlich mindestens ein Siliconöl. Zu denen Silikonölen zählen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Besonders bevorzugt sind flüchtige lineare Siliconöle, insbesondere Hexamethyldisiloxan (L₂), Octamethyltrisiloxan (L₃), Decamethyltetrasiloxan (L₄), Dodecamethylpentasiloxan (L₅), sowie beliebige Zweier-, Dreier und Vierermischungen aus L₂, L₃, L₄ und/oder L₅, wie sie z. B. in den Handelsprodukten Dow Corning 2-1184 Fluid, Dow Corning® 200 (0,65 cSt) und Dow Corning® 200 (1,5 cSt) von Dow Corning enthalten sind, wobei sich die Werte der kinematischen Viskosität auf eine Temperatur von 25°C beziehen.
Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte kosmetische Zubereitungen a) weiterhin mindestens ein nichtflüchtiges kosmetisches Öl, ausgewählt aus nichtflüchtigen Siliconölen und nichtflüchtigen Nichtsiliconölen, enthalten.
Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 bis 100 cSt, bevorzugt 5 bis 50 cSt oder auch 5 bis 10 cSt, und Baysilon® 350 M (mit einer kinematischen Viskosität (25°C) von etwa 350 cSt).

Die Verwendung eines erfindungsgemäßen Aerosolschaums zur temporären Umformung keratinischer Fasern ist ein weiterer Gegenstand dieser Anmeldung.

### Beispiele:

Die Zusammensetzung einiger bevorzugter kosmetischer Aerosolzusammensetzungen kann den folgenden Tabellen entnommen werden (die Angaben beziehen sich jeweils auf Gew.-% AS der jeweiligen Komponente bezogen auf das Gesamtgewicht der Aerosolzusammensetzung, sofern nicht anders angegeben).

**Tabelle 1**

| | **A** | **B** | **C** | **D** | **E** |
|---|---|---|---|---|---|
| Butan | 1,0 - 40,0 | 1,0 - 40,0 | 1,0 - 40,0 | 1,0 - 40,0 | 1,0 - 40,0 |
| Propan | 1,0 - 40,0 | 1,0 - 40,0 | 1,0 - 40,0 | 1,0 - 40,0 | 1,0 - 40,0 |
| VP/VA-Copolymer (Luviskol VA 64® Powder) | 2,0 - 4,0 | 2,0 - 4,0 | 2,0 - 4,0 | | |
| VP/MethacrylamideNinyl Imidazole Copolymer (Luviset Clear®) | | | | 2,0 - 4,0 | 2,0 - 4,0 |
| PEG-3 Benzyl Ether Myristate (Crodamol STS-LQ®) | 0,5 - 3,0 | 0,5 - 3,0 | 0,5 - 3,0 | 0,5 - 3,0 | 0,5 - 3,0 |
| Polyquaternium-11 (Gafquat 755®) | 0,1 - 3,0 | | | 0,1 - 3,0 | |
| Polyquaternium-16 (Luviquat FC 550®) | | 0,1 - 3,0 | | | |
| Polyquaternium-44 (Luviquat Care®) | | | 0,1 - 3,0 | | 0,1 - 3,0 |
| Propylenglycol | 0,1 - 5,0 | 0,1 - 5,0 | 0,1 - 5,0 | 0,1 - 5,0 | 0,1 - 5,0 |
| Cetrimoniumchlorid | 0,1 - 2,0 | 0,1 - 2,0 | | 0,1 - 2,0 | |
| Steartrimoniumchlorid | | | 0,1 - 2,0 | | 0,1 - 2,0 |
| PEG-40 Hydrogenated Castor Oil | 0,1 - 2,0 | 0,1 - 2,0 | 0,1 - 2,0 | 0,1 - 2,0 | 0,1 - 2,0 |
| Panthenol | 0,1 - 1,0 | 0,1 - 1,0 | | 0,1 - 1,0 | |
| Konservierungsmittel (Natriumbenzoat, Phenoxyethanol) | q.s. | q.s. | q.s. | q.s. | q.s. |
| Säuren zur pH-Einstellung (Milchsäure, Zitronensäure) | q.s. | q.s. | q.s. | q.s. | q.s. |
| Wasser | ad 100 | ad 100 | ad 100 | ad 100 | ad 100 |

**Tabelle 2**

| | **A** | **B** | **C** | **D** | **E** |
|---|---|---|---|---|---|
| Butan | 1,5 - 15,0 | 1,5 - 15,0 | 1,5 - 15,0 | 1,5 - 15,0 | 1,5 - 15,0 |
| Propan | 1,5 - 15,0 | 1,5 - 15,0 | 1,5 - 15,0 | 1,5 - 15,0 | 1,5 - 15,0 |
| VP/VA-Copolymer (Luviskol VA 64® Powder) | 2,0 - 3,0 | 2,0 - 3,0 | 2,0 - 3,0 | | |
| VP/MethacrylamideNinyl Imidazole Copolymer (Luviset Clear®) | | | | 2,0 - 3,0 | 2,0 - 3,0 |
| PEG-3 Benzyl Ether Myristate (Crodamol STS-LQ®) | 0,5 - 1,5 | 0,5 - 2,0 | 0,5 - 1,5 | 0,5 - 1,5 | 0,5 - 2,0 |
| Polyquaternium-11 (Gafquat 755®) | 0,2 - 1,0 | | | 0,1 - 1,0 | |
| Polyquaternium-16 (Luviquat FC 550®) | | 0,2-1,0 | | | |
| Polyquaternium-44 (Luviquat Care®) | | | 0,1 - 1,0 | | 0,1 - 1,0 |
| Propylenglycol | 0,5 - 2,0 | 0,5 - 3,0 | 0,5 - 2,0 | 0,5 - 3,0 | 0,5 - 3,0 |
| Cetrimoniumchlorid | 0,2 - 1,0 | 0,2 - 1,0 | | 0,2 - 1,0 | |
| Steartrimoniumchlorid | | | 0,2 - 1,0 | | 0,2 - 1,0 |
| PEG-40 Hydrogenated Castor Oil | 0,2 - 1,0 | 0,2 - 1,0 | 0,2 - 1,0 | 0,2 - 1,0 | 0,2 - 1,0 |
| Panthenol | 0,1 - 0,5 | 0,1 - 0,5 | | 0,1 - 0,5 | |
| Konservierungsmittel (Natriumbenzoat, Phenoxyethanol) | q.s. | q.s. | q.s. | q.s. | q.s. |
| Säuren zur pH-Einstellung (Milchsäure, Zitronensäure) | q.s. | q.s. | q.s. | q.s. | q.s. |
| Wasser | ad 100 | ad 100 | ad 100 | ad 100 | ad 100 |

## Patentansprüche

1. Aerosolschaum, umfassend
a) mindestens ein Treibmittel und
b) mindestens eine kosmetische Zubereitung, enthaltend
(i) mindestens einen festigenden Wirkstoff und
(ii) mindestens einen alkoxylierten Carbonsäureester der nachfolgenden Formel (I) 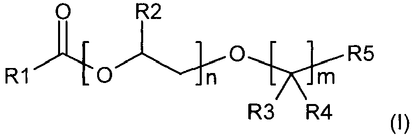 in der
- R1 für einen geradkettigen oder verzweigten, gesättigten oder ungesättigten Alkylrest mit 6 bis 24 Kohlenstoffatomen steht,
- R2 für Wasserstoff oder einen Alkylrest mit 1 bis 4 Kohlenstoffatomen steht,
- R3 oder R4 unabhängig voneinander für Wasserstoff oder einen Alkylrest mit 1 bis 4 Kohlenstoffatomen stehen,
- R5 für einen geradkettigen oder verzweigten, gesättigten oder ungesättigten Alkylrest mit 6 bis 24 Kohlenstoffatomen oder für einen Phenylrest steht, und
- n und m unabhängig voneinander für die Zahlen 0 oder 1 bis 20 stehen und
(iii) 50 bis 98 Gew.-% Wasser, bezogen auf das Gesamtgewicht des Aerosolschaums.

2. Aerosolschaum nach Anspruch 1, **dadurch gekennzeichnet, dass** die Zubereitung b) einen alkoxylierten Carbonsäureester gemäß Formel (I) enthält, in der
- R1 für einen geradkettigen, gesättigten Alkylrest mit 8 bis 18 Kohlenstoffatomen, bevorzugt für einen Decyl-, Lauryl-, Myristyl-, Palmityl- oder Stearylrest,
- R2 für einen Methylrest,
- R3 und R4 jeweils für Wasserstoff,
- R5 für einen Phenylrest,
- n für eine Zahl von 1 bis 10, bevorzugt von 2 bis 7, und
- m für die Zahlen 1 oder 2 steht.

3. Aerosolschaum nach einem der vorhergehenden Ansprüche, **dadurch gekennzeichnet, dass** die Zubereitung b) als festigenden Wirkstoff (i) mindestens ein Vinylpyrrolidon Copolymer enthält.

4. Aerosolschaum nach einem der vorherigen Ansprüche, **dadurch gekennzeichnet, dass** die Zubereitung b) als festigenden Wirkstoff (i) mindestens ein nichtionisches festigendes Polymer und mindestens ein kationisches festigendes Polymer enthält.

5. Aerosolschaum nach einem der vorherigen Ansprüche, **dadurch gekennzeichnet, dass** die Zubereitung b) als mindestens einen festigenden Wirkstoff (i) mindestens ein nichtionisches festigendes Polymer aus der Gruppe der Vinylpyrrolidon Copolymere und mindestens ein kationisches festigendes Polymer aus der Gruppe der Vinylpyrrolidon Copolymere enthält.

6. Aerosolschaum nach einem der vorherigen Ansprüche, **dadurch gekennzeichnet, dass** der Gewichtsanteil des festigenden Wirkstoffs (i) am Gesamtgewicht des Aerosolschaums 0,5 bis 12 Gew.-%, vorzugsweise 1,0 bis 10 Gew.-% und insbesondere 2,0 bis 8,0 Gew.-% beträgt.

7. Aerosolschaum nach einem der vorhergehenden Ansprüche, **dadurch gekennzeichnet, dass** der Gewichtsanteil des Carbonsäureesters gemäß Formel (I) am Gesamtgewicht des Aerosolschaums 0,1 bis 5,0 Gew.-%, vorzugsweise 0,2 bis 3,0 Gew.-% und insbesondere 0,3 bis 2,0 Gew.-% beträgt.

8. Aerosolschaum nach einem der vorherigen Ansprüche, **dadurch gekennzeichnet, dass** der Aerosolschaum bezogen auf sein Gesamtgewicht 60 bis 95 Gew.-% und insbesondere 70 bis 90 Gew.-% Wasser enthält.

9. Aerosolschaum nach einem der vorherigen Ansprüche, **dadurch gekennzeichnet, dass** das Treibmittel a) ausgewählt ist aus Propan und/oder Butan, wobei der gemeinsame Gewichtsanteil von Propan und/oder Butan am Gesamtgewicht des Aerosolschaums 1-99,5 Gew.-%, vorzugsweise 4 bis 50 Gew.-% beträgt.

10. Aerosolschaum nach einem der vorherigen Ansprüche, **dadurch gekennzeichnet, dass** die Zubereitung b) weiterhin mindestens ein kationisches Tensid, bevorzugt ausgewählt aus der Gruppe der Alkyltrimethylammoniumchloride, der Dialkyldimethylammoniumchloride und der Trialkylmethylammoniumchloride, in denen die langen Alkylketten bevorzugt 10 bis 18 Kohlenstoffatome aufweisen, enthält.

11. Aerosolschaum nach einem der vorherigen Ansprüche, **dadurch gekennzeichnet, dass** das mindestens eine kationische Tensid in einem Gewichtsanteil von 0,02 bis 5,0 Gew.-%, bevorzugt von 0,5 bis 3 Gew.-% und insbesondere von 0,1 bis 2 Gew.-%, bezogen auf das Gesamtgewicht des Aerosolschaums eingesetzt wird.

12. Aerosolschaum nach einem der vorherigen Ansprüche, **dadurch gekennzeichnet, dass** die Zubereitung b) weiterhin mindestens ein nichtionisches Tensid, vorzugsweise ausgewählt aus der Gruppe der alkoxylierten Fettalkohole und/oder der alkoxylierten Rizinusöle, enthält.

13. Verwendung eines Aerosolschaums nach einem der Ansprüche 1 bis 12 zur temporären Umformung keratinischer Fasern.

## Claims

1. An aerosol mousse, comprising
a) at least one propellant and
b) at least one cosmetic preparation, containing
(i) at least one setting active ingredient and
(ii) at least one alkoxylated carboxylic acid ester of the following formula (I) 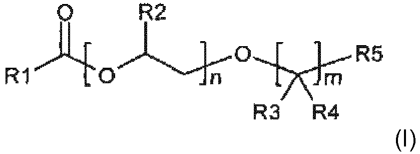 in which
- R1 represents a straight-chain or branched, saturated or unsaturated alkyl functional group having 6 to 24 carbon atoms,
- R2 represents hydrogen or an alkyl functional group having 1 to 4 carbon atoms,
- R3 or R4 represent, independently of one another, hydrogen or an alkyl functional group having 1 to 4 carbon atoms,
- R5 represents a straight-chain or branched, saturated or unsaturated alkyl functional group having 6 to 24 carbon atoms or a phenyl functional group, and
- n and m represent, independently of each other, the numbers 0 or 1 to 20 and
(iii) 50 to 98 wt.% water, based on the total weight of the aerosol mousse.

2. The aerosol mousse according to claim 1, **characterized in that** the preparation b) contains an alkoxylated carboxylic acid ester according to formula (I), in which
- R1 represents a straight-chain, saturated alkyl functional group having 8 to 18 carbon atoms, preferably a decyl, lauryl, myristyl, palmityl, or stearyl functional group,
- R2 represents a methyl functional group,
- R3 and R4 each represent hydrogen,
- R5 represents a phenyl functional group,
- n represents a number from 1 to 10, preferably from 2 to 7, and
- m represents the number 1 or 2.

3. The aerosol mousse according to one of the preceding claims, **characterized in that** the preparation b) contains at least one vinylpyrrolidone copolymer as the setting active ingredient (i).

4. The aerosol mousse according to one of the preceding claims, **characterized in that** the preparation b) contains at least one non-ionic setting polymer and at least one cationic setting polymer as the setting active ingredient (i).

5. The aerosol mousse according to one of the preceding claims, **characterized in that** the preparation b) contains at least one non-ionic setting polymer from the group of vinylpyrrolidone copolymers and at least one cationic setting polymer from the group of vinylpyrrolidone copolymers as at least one setting active ingredient (i).

6. The aerosol mousse according to one of the preceding claims, **characterized in that** the weight proportion of the setting active ingredient (i) with respect to the total weight of the aerosol mousse is 0.5 to 12 wt.%, preferably 1.0 to 10 wt.%, and in particular 2.0 to 8.0 wt.%.

7. The aerosol mousse according to one of the preceding claims, **characterized in that** the weight proportion of the carboxylic acid ester according to formula (I) with respect to the total weight of the aerosol mousse is 0.1 to 5.0 wt.%, preferably 0.2 to 3.0 wt.%, and in particular 0.3 to 2.0 wt.%.

8. The aerosol mousse according to one of the preceding claims, **characterized in that** the aerosol mousse contains, based on the total weight thereof, 60 to 95 wt.%, and in particular 70 to 90 wt.%, water.

9. The aerosol mousse according to one of the preceding claims, **characterized in that** the propellant a) is selected from propane and/or butane, the joint weight proportion of propane and/or butane with respect to the total weight of the aerosol mousse being 1 to 99.5 wt.%, preferably 4 to 50 wt.%.

10. The aerosol mousse according to one of the preceding claims, **characterized in that** the preparation b) also contains at least one cationic surfactant, preferably selected from the group of alkyltrimethylammonium chlorides, dialkyldimethylammonium chlorides, and trialkylmethylammonium chlorides, in which the long alkyl chains have preferably 10 to 18 carbon atoms.

11. The aerosol mousse according to one of the preceding claims, **characterized in that** the at least one cationic surfactant is used in a weight proportion of from 0.02 to 5.0 wt.%, preferably from 0.5 to 3 wt.%, and in particular from 0.1 to 2 wt.%, based on the total weight of the aerosol mousse.

12. The aerosol mousse according to one of the preceding claims, **characterized in that** the preparation b) also contains at least one non-ionic surfactant, preferably selected from the group of alkoxylated fatty alcohols and/or alkoxylated castor oils.

13. The use of an aerosol mousse according to one of claims 1 to 12 for temporarily shaping keratin fibers.

## Revendications

1. Mousse aérosol comprenant
a) au moins un agent propulseur, et
b) au moins une composition cosmétique contenant
(i) au moins un principe actif fixant, et
(ii) au moins un ester d'acide carboxylique alcoxylé répondant à la formule (I) suivante 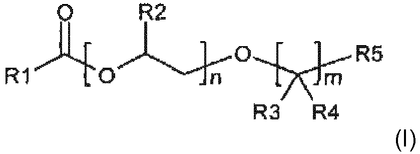 dans laquelle
R1 représente un radical alkyle linéaire ou ramifié, saturé ou insaturé, ayant 6 à 24 atomes de carbone,
- R2 représente un atome d'hydrogène ou un radical alkyle ayant 1 à 4 atomes de carbone,
- R3 ou R4 représentent indépendamment un atome d'hydrogène ou un radical alkyle ayant 1 à 4 atomes de carbone,
- R5 représente un radical alkyle linéaire ou ramifié, saturé ou insaturé ayant 6 à 24 atomes de carbone, ou représente un radical phényle, et
- n et m représentent indépendamment les nombres 0 ou 1 à 20, et
(iii) 50 à 98 % en poids d'eau par rapport au poids total de la mousse aérosol.

2. Mousse aérosol selon la revendication 1, **caractérisée en ce que** la composition b) contient un ester d'acide carboxylique alcoxylé selon la formule (I), dans laquelle
- R1 représente un radical alkyle linéaire saturé ayant 8 à 18 atomes de carbone, de préférence un radical décyle, lauryle, myristyle, palmityle ou stéaryle, R2 représente un radical méthyle,
- R3 et R4 représentent respectivement un atome d'hydrogène,
- R5 représente un radical phényle,
- n représente un nombre compris entre 1 et 10, de préférence entre 2 et 7, et
- m représente les nombres 1 ou 2.

3. Mousse aérosol selon l'une des revendications précédentes, **caractérisée en ce que** la composition b) contient au moins un copolymère de vinylpyrrolidone comme principe actif fixant (i).

4. Mousse aérosol selon l'une des revendications précédentes, **caractérisée en ce que** la composition b) contient au moins un polymère non ionique fixant et au moins un polymère cationique fixant comme principe actif fixant (i).

5. Mousse aérosol selon l'une des revendications précédentes, **caractérisée en ce que** la composition b) contient, comme au moins un principe actif de fixation (i), au moins un polymère non ionique fixant choisi dans le groupe constitué par les copolymères de vinylpyrrolidone et au moins un polymère cationique fixant choisi dans le groupe constitué par les copolymères de vinylpyrrolidone.

6. Mousse aérosol selon l'une des revendications précédentes, **caractérisée en ce que** la proportion en poids du principe actif fixant (i) par rapport au poids total de la mousse aérosol est de 0,5 à 12 % en poids, de préférence de 1,0 à 10 % en poids et en particulier de 2,0 à 8,0 % en poids.

7. Mousse aérosol selon l'une des revendications précédentes, **caractérisée en ce que** la proportion en poids de l'ester d'acide carboxylique selon la formule (I) par rapport au poids total de la mousse aérosol est de 0,1 à 5,0 % en poids, de préférence de 0,2 à 3,0 % en poids et en particulier de 0,3 à 2,0 % en poids.

8. Mousse aérosol selon l'une des revendications précédentes, **caractérisée en ce que** la mousse aérosol contient 60 à 95 % en poids d'eau par rapport à son poids total, et en particulier 70 à 90 % en poids.

9. Mousse aérosol selon l'une des revendications précédentes, **caractérisée en ce que** l'agent propulseur a) est choisi parmi le propane et/ou le butane, la proportion combinée en poids de propane et/ou de butane par rapport au poids total de la mousse aérosol étant de 1 à 99,5 % en poids, de préférence de 4 à 50 % en poids.

10. Mousse aérosol selon l'une des revendications précédentes, **caractérisée en ce que** la composition b) contient en outre au moins un tensioactif cationique, de préférence choisi dans le groupe constitué par les chlorures d'alkyltriméthylammonium, les chlorures de dialkyldiméthylammonium et les chlorures de trialkylméthylammonium, dans lesquels les longues chaînes alkyle présentent de préférence 10 à 18 atomes de carbone.

11. Mousse aérosol selon l'une des revendications précédentes, **caractérisée en ce que** l'au moins un tensioactif cationique est utilisé dans une proportion en poids comprise entre 0,02 et 5,0 % en poids, de préférence entre 0,5 et 3 % en poids et en particulier entre 0,1 et 2 % en poids par rapport au poids total de la mousse aérosol.

12. Mousse aérosol selon l'une des revendications précédentes, **caractérisée en ce que** la composition b) contient en outre au moins un tensioactif non ionique, de préférence choisi dans le groupe constitué par les alcools gras alcoxylés et/ou les huiles de ricin alcoxylées.

13. Utilisation d'une mousse aérosol selon l'une des revendications 1 à 12, destinée à la mise en forme temporaire de fibres kératiniques.
